(19) Europäisches Patentamt
European Patent Office
Office européen des brevets

(11) Publication number: 0 180 290 B1

(12) # EUROPEAN PATENT SPECIFICATION

(45) Date of publication of the patent specification: **28.10.87**

(51) Int. Cl.4: **C 07 C 59/88,** C 07 C 51/353 // A61K31/19

(21) Application number: **85304071.5**

(22) Date of filing: **10.06.85**

(54) **Omega-(2',4'-dihalobiphenylyl)oxo alkanoic acids and process for their preparation.**

(30) Priority: **31.08.84 CS 6586/84**

(43) Date of publication of application: **07.05.86 Bulletin 86/19**

(45) Publication of the grant of the patent: **28.10.87 Bulletin 87/44**

(84) Designated Contracting States: **AT BE CH DE FR GB IT LI SE**

(56) References cited:
**CH - A - 628 014**
**DE - A - 2 112 716**

(73) Proprietor: **SPOFA Spojené Podniky Pro Zdravotnickou Vyrobu, Husinecká 11 a, Prag 3 (CS)**

(72) Inventor: **Kuchar, Miroslav, CSc, No. 9 Cernokostelecka, Praha 10, (CS)**
Inventor: **Brunova, Bohumila, CSc, No. 3 Ostrovskeho, Praha 5 (CS)**
Inventor: **Grimova, Jaroslava, CSc, No. 2 Na Doubkove, Praha 5 (CS)**
Inventor: **Maturova, Eva, No. 2 Zvonkova, Praha 10 (CS)**

(74) Representative: **Wotherspoon, Graham et al, FITZPATRICKS 4 West Regent Street, Glasgow G2 1RS Scotland (GB)**

EP 0 180 290 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to omega-(2',4'-dihalobiphenyl) oxo alkanoic acids of the general formula I

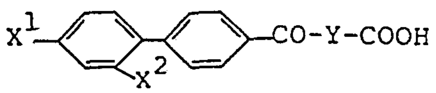 (I)

in which $X^1$ and $X^2$ each represent a fluorine or chlorine atom and Y represents a bivalent $-CH_2CH(CH_3)-$ or $-(CH_2)_3-$ chain, and to a process for the preparation thereof.

Certain 4-aryl-4-oxo butanoic acids are already known to possess antiinflammatory properties (Belgian Patent No. 772,804): thus 4-biphenylyl-4-oxo butanoic acid (fenbufen) has found clinical use in the treatment of reheumatoid diseases. Similarly, also monohalo derivatives of 4-biphenylyl-4-oxo-2-methylbutanoic acide (Swiss Patent No. 628,014) have antiinflammatory activity of short duration.

It has recently been surprisingly observed that a ditopic substitution by a chlorine or fluorine atom on the outer aromatic ring, optionally with a modification of the aliphatic chain connecting the oxo and the carboxyl group, produces, at substantially no increase in toxicity, a remarkable prolongation of the antiinflammatory effect to more than 24 hours. A remarkably protracted antiinflammatory effect was observed especially in 4-(2',4'-difluorobiphenylyl)-2-methyl-4-oxo butanoic and 4-(2',4'-dichlorobiphenylyl)-2-methyl-4-oxo butanoic acid.

The antiinflammatory activity and short-term toxicity assay results for two typical compounds of the invention are given in the Table below. The compounds under study showed, in both of the standard assay methods used, remarkable antiinflammatory effects which persisted, at a dosage level of 10 to 25 mg/kg p.o., for over 24 or event 48 hours. For sake of comparison, the corresponding data for fenbufen and 4-(4'-fluorbiphenylyl)-2-methyl-4-oxo butanoic acid are also tabulated. These two reference compounds showed a somewhat protracted antiinflammatory effect only upon administration of doses as high as 100 mg/kg p.o. and their action lasted for a shorter time period than that of the tested compounds of the invention administered at a substantially lower dosage level.

*Table*

Pharmacological evaluation of the acute toxicity ($LD_{50}$) and antiinflammatory activity as per the carrageenin oedema (CE)[a] and Freund adjuvans oedema (FA)[b] inhibition

| Compound[x] ($X^1$, $X^2$) | $LD_{50}$ | Dose mg/kg p.o. | CE inhibition[c] % after 1 h | 24 h | 48 h | Dose mg/kg p.o. | FA inhibition[c] % after 1 h | 24 h |
|---|---|---|---|---|---|---|---|---|
| I (F,F) | 450 | 25 | 27 | 54 | 58 | 10 | 37 | 38 |
| | | 100 | 35 | 73 | 58 | 25 | 40 | 46 |
| | | | | | | 100 | 45 | 55 |
| II (Cl,Cl) | 500 | 25 | 30 | 61 | 42 | 25 | 56 | 32 |
| | | 100 | 44 | 66 | 45 | 50 | 53 | 46 |
| Ir (F,H) | 500 | 25 | 16[n] | 21 | — | 10 | 37 | 31 |
| | | 100 | 33 | 34 | — | 20 | 42 | 39 |
| IIr (fenbufen) | 600 | 25 | 40 | 14[n] | — | 10 | 27 | 8[n] |
| | | 100 | 46 | 38 | 0 | 25 | 31 | 9[n] |
| | | | | | | 100 | 54 | 7[n] |

Please note:

a – assay technique used: Winter J., Proc. Soc. Exptl. Biol. Med. *111* (1962) 544, single oral doses as tabulated, oedema size evaluated 1 h, 24 h and 48 h after administration,

b – assay technique used: Horáková Z. and Grimová J., Českosl. Fysiol. *17* (1968) 137, dosage as above (cf. *a*), evaluation 1 h and 24 h after dosage,

c – tabulated values – except those indexed[n] – are statistically significant ($p \leqslant 0.05$),

x – general formula I, Y stands for a $-CH_2CH(CH_3)-$ chain, I and II – compounds of the application (examples 1 and 2, resp.)
Ir and IIr (fenbufen) – reference compounds for comparison.

The compounds of the invention can be formulated into therapeutic dosage forms which contain the active substance in a mixture with liquid and/or solid physiologically benign auxiliaries which are common in the manufacture of pharmaceutical preparations.

The exact dosage depends on the particular nature and disease pattern, but the general dosage scheme can be derived from the tabulated potency data: principally, oral doses at the same level as for the mentioned reference compounds, or even lower, give a comparable therapeutical effect lasting over a substantially longer period of time.

The compounds of the general formula I are advantageously prepared by reacting a 2,4-dihalo biphenyl of the general formula II

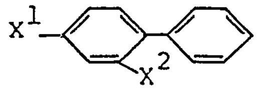

(II)

in which $X^1$ and $X^2$ have the same meaning as in formula I with a dicarboxylic acid anhydride of the general formula III

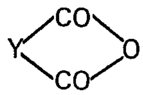

(III)

in which Y has the same meaning as in formula I, in an inert organic solvent, preferably 1,2-dichloroethane, nitromethane or carbon disulfide, in the presence of Friedel-Crafts type catalyst such as anhydrous aluminium chloride. The reaction may be carried out at a temperature of from 0 to 50° C, preferably at 10 to 20° C.

The starting materials required for the preparation are known substances available by methods described in the pertinent literature.

The invention will now be described by, way of illustration, in the following Examples.

*Example 1*

*4-(2',4'-Difluorobiphenylyl)-2-methyl-4-oxo butanoic acid*

Anhydrous aluminium chloride (33.2 g, 0.25 mole) and methylsuccinic acid anhydride (21.6 g, 0.188 mole) were dissolved in 1,2-dichloroethane (190 ml). The solution was cooled to 15° C and treated, with stirring and cooling, with a solution of 2,4-difluorobiphenyl (47.5 g, 0.25 mole) in the same solvent (50 ml) at such a rate that the temperature of the reaction mixture did not exceed 20° C. The mixture was then stirred for an additional 3 hours at 20° C and 1 hour at 40 to 45° C, cooled to 20° C and poured slowly, with stirring, into a mixture of ice (500 g) and concentrated hydrochloric acid (250 ml). The precipitate was collected on a filter, and the organic layer of the filtrate was separated and washed with a 5% aqueous sodium hydroxide solution (4 × 100 ml). The alkaline extracts were combined and the precipitate was dissolved in the extracts. The moderately turbid solution thus obtained was filtered with active carbon and the clear filtrate was acidified with 50% (approximately) sulfuric acid to pH 1. The precipitated crude product was separated, washed with water and purified by crystallization from 65% acetic acid (160 ml) to give the title compound (240 g, 42% of theory) melting at 150 to 152° C.

*Example 2*

*4-(2',4'-Dichlorobiphenylyl)-2-methyl-4-oxo butanoic acid*

A solution of 2,4-dichlorobiphenyl (33.4 g, 0,15 mole) and methylsuccinic acid anhydride (18.0 g, 0.15 mole) in carbon disulfite (100 ml) was cooled to 10° C and treated portionwise, with stirring, with anhydrous aluminium chloride (24.0 g, 0.18 mole): the temperature should not exceed 15° C. The reaction mixture was stirred for an additional 4 hours at 20° C and then decomposed by pouring into a mixture of water (350 ml) and concentrated hydrochloric acid (175 ml). The carbon disulfide was distilled off and the residue was washed with 1,2-dichloroethane (2 × 200 ml and 2 × 100 ml). The extracts were combined and washed with a 5% aqueous sodium hydroxide solution (5 × 75 ml) and water (1 × 75 ml). The alkaline extracts and the aqueous washing were combined, filtrered with active carbon and the clear filtrate obtained was acidified with 50% sulfuric acid to a strongly acidic reaction. The precipitate was separated, washed with water and purified by crystallization from 65% acetic acid (95 ml). The yield is 19.3 g (38.0% of theory) of the title compound melting at 155 to 157° C.

As described above, said 2,4-difluorobiphenyl and glutaric acid anhydride give 5-(2',4'-difluorobiphenylyl)-5-oxo pentanoic acid, m.p. 178-180° C (18.5% of theory after crystallization from 65% acetic acid).

**Claims** for the Contracting States BE, CH, DE, FR, GB, IT, LI, SE

1. Omega-(2',4'-Dihalobiphenylyl)oxo alkanoic acids of the general formula I

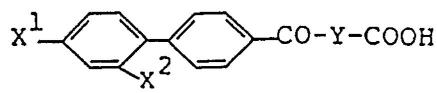

(I)

in which $X^1$ and $X^2$ each represent a fluorine or chlorine atom and Y represents a bivalent $-CH_2CH(CH_3)-$ or $-(CH_2)_3-$ chain.

2. 4-(2',4'-Difluorobiphenylyl)-2-methyl-4-oxo butanoic acid.

3. 4-(2',4'-Dichlorobiphenylyl)-2-methyl-4-oxo butanoic acid.

4. A process for the preparation of an omega-(2',4'-dihalobiphenylyl)oxo alkanoic acid of the general formula I

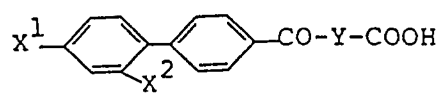

(I)

in which $X^1$ and $X^2$ each represent a fluorine or chlorine atom and Y represents a bivalent $-CH_2CH(CH_3)-$ or $-(CH_2)_3-$ chain, said process comprising reacting a 2,4-dihalo biphenyl of the general formula II

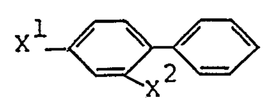

(II)

in which $X^1$ and $X^2$ have the same meaning as in formula I, with a dicarboxylic acid anhydride of the general formula III

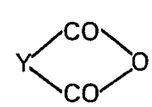

(III)

in which y has the same meaning as in formula I.

5. A process according to claim 4 for the preparation of 4-(2',4'-difluorobiphenylyl)-2-methyl-4-oxo butanoic acid, comprising reacting 2,4-difluorobiphenyl with methylsuccinic acid anhydride.

6. A process according to claim 4 for the preparation of 4-(2',4'-dichlorobiphenylyl)-2-methyl-4-oxo butanoic acid, comprising reacting 2,4-dichlorobiphenyl with methylsuccinic acid anhydride.

7. A process according to any of claims 4 to 6 which comprises combining said reactants in an inert organic solvent in the presence of anhydrous aluminium chloride at a temperature of from 0 to 50° C.

**Claims** for the Contracting State : AT

1. A process for the preparation of an omega-(2,4-dihalobiphenylyl)oxo alkanoic acid of the general formula I

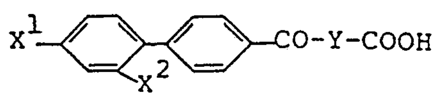
(I)

in which $X^1$ and $X^2$ each represent a fluorine or chlorine atom and Y represents a bivalent $-CH_2CH(CH_3)-$ or $-(CH_2)_3-$ chain, said process comprising reacting a 2,4-dihalo biphenyl of the general formula II

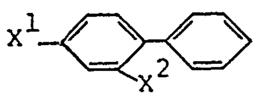
(II)

in which $X^1$ and $X^2$ have the same meaning as in formula I, with a dicarboxylic acid anhydride of the general formula III

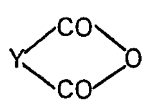
(III)

in which Y has the same meaning as in formula I.

2. A process according to claim 1, for the preparation of 4-(2,4-difluorobiphenylyl)-2-methyl-4-oxo butanoic acid, comprising reacting 2,4-difluorobiphenyl with methylsuccinic acid anhydride.

3. A process according to claim 1 for the preparation of 4-(2,4-dichlorobiphenylyl)-2-methyl-4-oxo butanoic acid, comprising reacting 2,4-dichlorobiphenyl with methylsuccinic acid anhydride.

4. A process according to any of claims 1 to 3, which comprises combining said reactants in an inert organic solvent in the presence of anhydrous aluminium chloride at a temperature of from 0 to 50° C.

**Patentansprüche** für die Vertragsstaaten Be, CH, DE, FR, GB, IT, LI und SE

1. Omega-(2',4'-Dihalogenbiphenylyl)oxoalkansäuren der allgemeinen Formel (I)

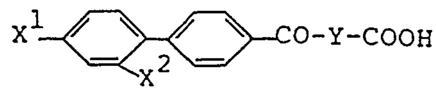
(I)

worin $X_1$ und $X_2$ jeweils ein Fluor- oder Chloratom bedeuten und Y eine divalente $-CH_2CH(CH_3)-$ oder $-(CH_2)_3$-Kette bedeutet.

2. 4-(2',4'-Difluorbiphenylyl)-2-methyl-4-oxobutansäure.

3. 4-(2',4'-Dichlorbiphenylyl)-2-methyl-4-oxobutansäure.

4. Verfahren zur Herstellung einer Omega-(2',4'-Dihalogenbiphenylyl)oxoalkansäure der allgemeinen Formel (I)

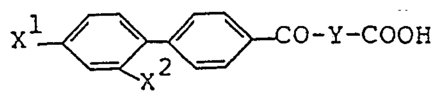
(I)

worin $X_1$ und $X_2$ jeweils ein Fluor- oder Chloratom bedeuten und Y eine divalente $-CH_2CH(CH_3)-$ oder $-(CH_2)_3$-Kette bedeutet, dadurch gekennzeichnet, dass ein 2,4-Dihalogenbiphenyl der allgemeinen Formel (II)

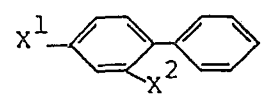
(II)

in welcher $X_1$ und $X_2$ die gleiche Bedeutung wie in der Formel I besitzen, mit einem Dicarbonsäureanhydrid der allgemeinen Formel (III)

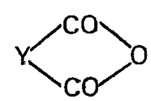
(III)

in welcher Y die gleiche Bedeutung wie in der Formel I hat, umgesetzt wird.

5. Verfahren nach Anspruch 4, zur Herstellung von 4-(2',4'-Difluorbiphenylyl)-2-methyl-4-oxobutansäure, dadurch gekennzeichnet, dass 2,4-Difluorbiphenyl mit Methylsuccinsäureanhydrid umgesetzt wird.

6. Verfahren nach Anspruch 4, zur Herstellung von 4-(2',4'-Dichlortriphenylyl)-2-methyl-4-oxobutansäure, dadurch gekennzeichnet, dass 2,4-Dichlorbiphenyl mit Methylsuccinsäureanhydrid umgesetzt wird.

7. Verfahren nach einem der Ansprüche 4 bis 6, dadurch gekennzeichnet, dass die Reaktanden in einem inerten organischen Lösungsmittel in Gegenwart von wasserfreiem Aluminiumchlorid bei Temperaturen zwischen 0 und 50° C eingesetzt werden.

**Patentansprüche** für den Vertragsstaat: AT

1. Verfahren zur Herstellung einer Omega-(2',4')-Dihalogenbiphenylyl)oxoalkansäure der allgemeinen Formel (I)

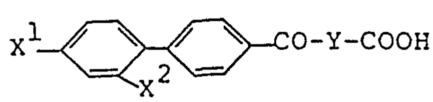
(I)

worin $X_1$ und $X_2$ jeweils ein Fluor- oder Chloratom bedeuten und Y eine divalente $-CH_2CH(CH_3)-$

oder $-(CH_2)_3$-Kette bedeutet, dadurch gekennzeichnet, dass ein 2,4-Dihalogenbiphenyl der allgemeinen Formel (II)

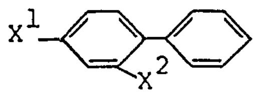

(II)

in welcher $X_1$ und $X_2$ die gleiche Bedeutung wie in der Formel I besitzen, mit einem Dicarbonsäureanhydrid der allgemeinen Formel (III)

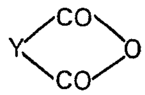

(III)

in welcher Y die gleiche Bedeutung wie in der Formel I hat, umgesetzt wird.

2. Verfahren nach Anspruch 1, zur Herstellung von 4-(2',4'-Difluorbiphenylyl)-2-methyl-4-oxobutansäure, dadurch gekennzeichnet, dass 2,4-Difluorbiphenyl mit Methylsuccinsäureanhydrid umgesetzt wird.

3. Verfahren nach Anspruch 1, zur Herstellung von 4-(2',4'-Dichlortriphenylyl)-2-methyl-4-oxobutansäure, dadurch gekennzeichnet, dass 2,4-Dichlorbiphenyl mit Methylsuccinsäureanhydrid umgesetzt wird.

4. Verfahren nach einem der Ansprüche 1 bis 3, dadurch gekennzeichnet, dass die Reaktanden in einem inerten organischen Lösungsmittel in Gegenwart von wasserfreiem Aluminiumchlorid bei Temperaturen zwischen 0 und 50° C eingesetzt werden.

**Revendications** pour les Etats Contractants BE, CH, DE, FR, GB, IT, LI et SE

1. Acides oméga-2',4'-dihalogénobiphénylyl)-oxoalcanoïques de formule générale I

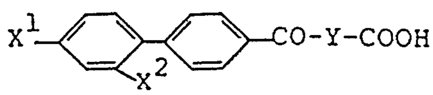

(I)

dans laquelle $X^1$ et $X^2$ représentent chacun un atome de fluor ou de chlore et Y représente un enchaînement $-CH_2CH(CH_3)-$ ou $-(CH_2)_3-$ bivalent.

2. Acide 4-(2',4'-difluorobiphénylyl)-2-méthyl-4-oxobutanoïque.

3. Acide 4-(2',4'-dichlorobiphénylyl)-2-méthyl-4-oxobutanoïque.

4. Procédé pour la préparation d'un acide oméga-(2',4'-dihalogénobiphénylyl)-oxo-alcanoïque de formule générale I

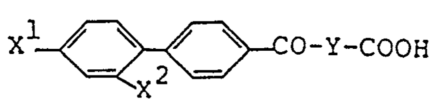

(I)

dans laquelle $X^1$ et $X^2$ représentent chacun un atome de fluor ou de chlore, et Y représente un enchaînement bivalent $-CH_2CH(CH_3)-$ ou $-(CH_2)_3-$, ledit procédé consistant à faire réagir un 2,4-dihalogénobiphényle de formule générale II

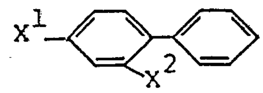

(II)

dans laquelle $X^1$ et $X^2$ ont les mêmes significations que dans la formule I, avec un anhydride d'acide dicarboxylique de formule générale III

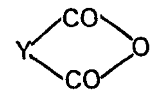

(III)

dans laquelle Y a la même signification que dans la formule I.

5. Procédé selon la revendication 4, pour la préparation de l'acide 4-(2',4'-difluorobiphénylyl)-2-méthyl-4-oxobutanoïque, consistant à faire réagir le 2,4-difluorobiphényle avec l'anhydride méthylsuccinique.

6. Procédé selon la revendication 4 pour la préparation de l'acide 4-(2',4'-dichlorobiphénylyl)-2-méthyl-4-oxobutanoïque, consistant à faire réagir le 2,4-dichlorobiphényle avec l'anhydride méthylsuccinique.

7. Procédé selon l'une des revendications 4 à 6, qui consiste à combiner lesdits réactifs, dans un solvant organique inerte, en présence de chlorure d'aluminium anhydre à une température de 0 à 50° C.

**Revendications** pour l'Etat Contractant: AT

1. Procédé pour la préparation d'un acide oméga-(2',4'-dihalogénobiphénylyl)oxo-alcanoïque de formule générale I

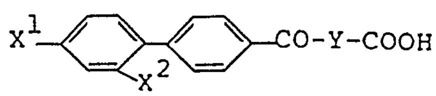

(I)

dans laquelle $X^1$ et $X^2$ représentent chacun un atome de fluor ou de chlore et Y représente un enchaînement bivalent $-CH_2CH(CH_3)-$ ou $-(CH_2)_3-$, ledit procédé consistant à faire réagir un 2,4-dihalogénobiphényle de formule générale II

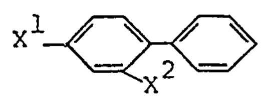

(II)

dans laquelle $X^1$ et $X^2$ ont les significations données dans la formule I, sur un anhydride d'acide dicarboxylique de formule générale III

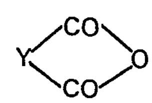

(III)

dans laquelle Y a la même signification que dans la formule I.

2. Procédé selon la revendication 1, pour la préparation de l'acide 4-(2',4'-difluorobiphénylyl)-2-méthyl-4-oxobutanoïque, consistant à faire réagir le 2,4-difluorobiphényle avec l'anhydride méthylsuccinique.

3. Procédé selon la revendication 1 pour la préparation de l'acide 4-(2',4'-dichlorobiphénylyl)-2-méthyl-4-oxobutanoïque, consistant à faire réagir le 2,4-dichlorobiphényle avec l'anhydride méthylsuccinique.

4. Procédé selon l'une des revendications 1 à 3, qui consiste à combiner lesdits réactifs dans un solvant organique inerte, en présence de chlorure d'aluminium anhydre à une température de 0 à 50° C.